# EUROPEAN PATENT SPECIFICATION

(11) **EP 2 535 328 B1**
(45) Date of publication and mention of the grant of the patent: **24.08.2016**
(21) Application number: 11742315.2
(22) Date of filing: 10.02.2011
(51) Int. Cl.: C07C 317/22, C09K 3/00, H01M 4/131, H01M 4/133, H01M 14/00, C07D 213/64, H01M 8/06, H01M 10/0565, H01M 10/052, H01M 4/13, H01M 4/137, H01G 9/20, H01G 11/50, H01M 8/0668

(54) **FLUOROALKANE DERIVATIVE, GELLING AGENT AND GEL COMPOSITION**
FLUORALKANDERIVAT, GELIERUNGSMITTEL UND GELZUSAMMENSETZUNG
DÉRIVÉ ALCANE FLUORÉ, AGENT GÉLIFIANT ET COMPOSITION DE GEL

(30) Priority: 12.02.2010 JP 2010029396
(43) Date of publication of application: 19.12.2012
(73) Proprietor: Asahi Kasei E-Materials Corporation, Chiyoda-ku Tokyo 101-8101 (JP); Yamaguchi University, Yamaguchi 753-8511 (JP)
(72) Inventor: OHASHI, Asami, Tokyo 101-8101 (JP); OKAMOTO, Hiroaki, Ube-shi Yamaguchi 755-8611 (JP); MORITA, Yuki, Ube-shi Yamaguchi 755-8611 (JP)
(74) Representative: D Young & Co LLP
(86) International application number: PCT/JP2011/052908
(87) International publication number: WO 2011/099572

(56) References cited:
- WO-A1-91/08198
- WO-A1-95/27692
- WO-A1-2005/019378
- WO-A1-2009/078268
- WO-A1-2010/095572
- WO-A1-2010/143658
- LAP-TAK A. CHENG ET AL: "Quadratic hyperpolarizabilities of fluorinated sulfonyl and carbonyl aromatics: optimization of nonlinearity and transparency trade-off", PROCEEDINGS OF SPIE, vol. 1337, 1 December 1990 (1990-12-01), pages 203-214, XP055053380, ISSN: 0277-786X, DOI: 10.1117/12.22969
- DATABASE CA [Online] CHEMICAL ABSTRACTS SERVICE, COLUMBUS, OHIO, US; 1991, TAM, WILSON ET AL: "Donor- and acceptor-substituted organic and organometallic compounds. Second-order nonlinear optical properties", XP002692142, retrieved from STN Database accession no. 1991:502156 & TAM, WILSON ET AL: "Donor- and acceptor-substituted organic and organometallic compounds. Second-order nonlinear optical properties", ACS SYMPOSIUM SERIES , 455(MATER. NONLINEAR OPT.), 158-69 CODEN: ACSMC8; ISSN: 0097-6156, 1991,
- ROBERT D. MILLER ET AL.: 'Substituted Azole Derivatives as Nonlinear Optical Chromophores' CHEMISTRY OF MATERIALS vol. 6, no. 7, 1994, pages 1023 - 1032, XP002028006
- KAZUNORI HIRABAYASHI ET AL.: 'A New Transformation of Silanols. Palladium-Catalyzed Cross-Coupling with Organic Halides in the Presence of Silver(I) Oxide' ORGANIC LETTERS vol. 1, no. 2, 1999, pages 299 - 301, XP008167249

## Description

### Technical Field

The present invention relates an electrode for an electrochemical device comprising a fluoroalkane derivative and use of a composition comprising a fluoroalkane derivative for sorbing carbon dioxide.

### Background Art

Gelling agents have been conventionally used for the purposes of solidifying liquid substances, that is, gelatinously solidifying them, or thickening liquid substances in various industrial fields (for example, coating, cosmetic, pharmaceutical and medical, spilled petroleum-treating, electronic and optical, and environmental fields).

These gelling agents include ones to gelatinize (guasi-solidify) water, and gelatinize (guasi-solidify) nonaqueous solvents, and solutions containing mainly the nonaqueous solvent, and the like. The structures of the gelling agents may be roughly classified into a high-molecular weight type and a low-molecular weight type. The high-molecular weight type gelling agents are used mainly for gelatinizing nonaqueous solvents, and have a feature that while a high-molecular weight polymer having lipophilicity takes oils in entangled molecules thereof and is swelled with the oils, the swelled polymer holds a solid state. By contrast, many of low-molecular weight type gelling agents contain hydrogen bonding functional groups (for example, an amino group, an amido group and a urea group) in molecules thereof, and have a feature that water and/or nonaqueous solvents are gelatinized through hydrogen bonds (see, for example, Patent Literature 1). Low-molecular weight type gelling agents are common as gelling agents of water, but the development as gelling agents of nonaqueous solvents is relatively slow.

Further, low molecular weight type gelling agents having no hydrogen-bonding groups are disclosed, for example, in Patent Literature 2 and Non-Patent Literature 1, but examples thereof are very few.

In addition, WO 2010/095572 discloses an electrolyte solution for a lithium ion secondary battery and a lithium ion secondary battery. WO 2010/143658 discloses an electrolyte solution and a lithium ion secondary battery utilizing the same WO 2009/078268 discloses compounds for use in batteries.

### Citation List

### Patent Literature

Patent Literature 1: Japanese Patent Application Laid-Open No. 8-231942
Patent Literature 2: International Publication No. WO 2009/78268

### Non Patent Literature

Non Patent Literature 1: J. Fluorine. Chem. 111, 47-58 (2001)

### Summary of Invention

### Technical Problem

Conventional gelling agents to gelatinize nonaqueous solvents generally need to be used in a large amount, for example, in about 10% to a solution and additionally has a tendency of changing to a sol and returning to a liquid state at a relatively low temperature, for example, about 30 to 40°C. Use of a large amount of a gelling agent to gelatinize a solvent (water or a nonaqueous solvent) is not only economically disadvantageous, but also means that the mixing amount of foreign matters into a solvent to be gelatinized becomes large; and in some cases of utilizing a gelatinized solvent, the influence of a gelling agent as an impurity cannot be disregarded. If the upper limit of the gelling temperature is low, due to a small rise in temperature, the solvent cannot hold the gel state, and may be fluidized and causes liquid leakage and the like in some cases. Then, the development of a gelling agent which can hold a gel state in a smaller amount of the gelling agent and up to a relatively high temperature is demanded.

In addition, Gelling agents, which can gelatinize nonaqueous solvents and systems in which strong hydrogen bonds cannot be present, are broadly demanded due to weak hydrogen bondability of the gelling agents.

However, conventional gelling agents have various types of problems, such as that can gelatinize few types of solvents, and have a drawback in stability of gels; and gelatinization of nonaqueous solvents needs a relatively large amount of a gelling agent.

### Solution to Problem

Described is a novel fluoroalkane derivative capable of gelatinizing or solidifying various types of nonaqueous solvents in a small amount of the addition, a gelling agent comprising the compound and a gelatinous composition comprising the gelling agent, as well as an electrode, an electrolyte solution for a dye-sensitized solar battery and a carbon dioxide-sorbing composition comprising the novel fluoroalkane derivative.

That is, the described fluoroalkane derivative is as follows.
[1] A fluoroalkane derivative represented by the following general formula (1):

   R-SO₂Ar-O-R¹ (1)

   wherein Ar denotes a substituted or nonsubstituted divalent aromatic group having 8 to 30 atoms of nucleus(es), R¹ denotes a saturated or unsaturated monovalent hydrocarbon group having 1 to 20 carbon atoms, and R denotes a saturated or unsaturated monovalent hydrocarbon group having a perfluoroalkyl group and having 2 to 22 carbon atoms.
[2] The fluoroalkane derivative according to [1], wherein the R is a group represented by the following general formula (2):

   CₘF₂ₘ₊₁CₚH₂ₚ (2)

   wherein m denotes a natural number of 2 to 16, and p denotes an integer of 0 to 6.
[3] The fluoroalkane derivative according to [1] or [2], wherein the Ar is a condensed ring having one or more aromatic hydrocarbon rings, or a group having a plurality of aromatic rings bonded to each other by a single bond, one or more of the aromatic rings being an aromatic hydrocarbon ring.
[4] The fluoroalkane derivative according to any one of [1] to [3], wherein the Ar is a biphenylene group.
[5] A gelling agent comprising a fluoroalkane derivative according to any one of [1] to [4].
[6] A gelatinous composition comprising a gelling agent according to [5] and an organic solvent.
[8] An electrolyte solution for a dye-sensitized solar battery, comprising a fluoroalkane derivative according to any one of [1] to [4].
[9] A carbon dioxide-sorbing composition comprising a fluoroalkane derivative according to any one of [1] to [4] and an ionic liquid.

The present invention provides an electrode for an electrochemical device, comprising a fluoroalkane derivative represented by the following general formula (1):

R-SO₂-Ar-O-R¹ (1)

wherein Ar denotes a substituted or nonsubstituted divalent aromatic group having 8 to 30 atoms of the nucleus(es), R¹ denotes a saturated or unsaturated monovalent hydrocarbon group having 1 to 20 carbon atoms, and R denotes a saturated or unsaturated monovalent hydrocarbon group having a perfluoroalkyl group and having 2 to 22 carbon atoms, wherein the R is a group represented by the following general formula (2):

CₘF₂ₘ₊₁CₚH₂ₚ- (2)

wherein m denotes a natural number of 2 to 16, and p denotes an integer of 2 to 4.

The present invention further provides the electrode for an electrochemical device according to claim 1, wherein the Ar is a condensed ring having one or more aromatic hydrocarbon rings, or a group having a plurality of aromatic rings bonded to each other by a single bond(s), one or more of the aromatic rings being an aromatic hydrocarbon ring.

### Advantageous Effect of Invention

It is possible to provide a fluoroalkane derivative capable of gelatinizing or solidifying various types of nonaqueous solvents in a small amount of the addition, a gelling agent comprising the compound, and a gelatinous composition comprising the gelling agent, as well as an electrode, an electrolyte solution for a dye-sensitized solar battery, and a carbon dioxide-sorbing composition comprising the fluoroalkane derivative.

### Description of Embodiment

Hereinafter, the embodiment according to the present invention (hereinafter, referred to simply as "the present embodiment") will be described in detail. A fluoroalkane derivative used in the present invention has an alkylsulfonyl group having a perfluoroalkyl group and a hydrocarbonoxy group, and is a compound represented by the general formula (1) shown above (hereinafter described as a "compound (1)"), in which R in formula (1) is a group represented by the general formula (2) shown above (hereinafter described as a "compound (2)")).

In general formula (1), Ar denotes a substituted or nonsubstituted divalent aromatic group having 8 to 30 atoms of the nucleus(es). The divalent aromatic group is a cyclic divalent group exhibiting so-called "aromaticity." " The divalent aromatic group may be a carbocyclic group or a heterocyclic group. These divalent aromatic groups may be ones substituted with a substituent or may be nonsubstituted ones, which are not substituted. The substituent of the divalent aromatic group is preferably selected from the viewpoint of easily permitting the introduction of a perfluoroalkyl(oligomethylene)thio group and the introduction of a hydrocarbonoxy group as described later.

The carbocyclic group has 10 to 30 atoms of the nucleus(es) and may be substituted with a substituent, or may be a nonsubstituted one, which is not substituted. Specific examples thereof include divalent groups having a nucleus(es) represented by a biphenylene group, a terphenylene group, a naphthylene group, an anthranylene group, a phenanthrylene group, a pyrenylene group, a chrysenylene group, and a fluoranthenylene group. In addition, the carbocyclic group may have two or more divalent groups described above (which may be identical to or different from each other) in the range of 10 to 30 atoms of the nucleus(es).

The heterocyclic group has 8 to 30 atoms of the nucleus(es) and examples thereof include divalent groups having two or more nucleuses (which may be identical to or different from each other) represented by a furanylene group, a thiophenylene group, a triazolene group, an oxadiazolene group, a pyridylene group, and a pyrimidylene group in the range of 8 to 30 atoms of the nucleus(es).

Further, Ar may be a group having both of the carbocyclic group and the heterocyclic group described above in the range of 8 to 30 atoms of the nucleus(es).

Since Ar having 8 or more atoms of the nucleus(es) exhibits a high gelling power and raises the versatility of selecting the structures of R and R¹ and the number of carbon atoms. In addition, raw materials of the compound (1) in which Ar has 30 or less atoms of the nucleus(es) are easily available, and the synthesis is also easy.

Among these, from the viewpoint of the gelling power and the synthesis easiness, the divalent aromatic group is preferably a condensed ring having one or more substituted or nonsubstituted aromatic hydrocarbon rings (more preferably benzene rings), or a group having a plurality of aromatic rings bonded to each other by a single bond(s) and one or more of the aromatic rings being an aromatic hydrocarbon ring (more preferably benzene rings). The divalent aromatic group is further preferably a substituted or nonsubstituted biphenylene group, terphenylene group, naphthylene group, anthranylene group, and phenylene pyridylene group (-Ph-Py-, wherein Ph denotes a benzene ring, and Py denotes a pyridine ring), and most preferably a biphenylene group. In addition, examples of the substituent described above include alkyl groups represented by a methyl group and an ethyl group, and a halogen atom. In the present description, "an aromatic ring" may be carbocyclic or heterocyclic.

R¹ denotes a saturated or unsaturated monovalent hydrocarbon group having 1 to 20 carbon atoms, and may be an aliphatic hydrocarbon group, and may further have an aromatic hydrocarbon group. In the case where the hydrocarbon group is a monovalent aliphatic hydrocarbon group, the hydrocarbon group may be branched or non-branched. Further in the case where the monovalent hydrocarbon group has an aromatic hydrocarbon group, the aromatic hydrocarbon group may further have a substituent, or no substituent. However, the monovalent hydrocarbon group, in order that a compound (1) is dissolved in a nonaqueous solvent, which is then gelatinized, is preferably a hydrocarbon group, capable of dissolving the compound (1) in the nonaqueous solvent, such as an arylalkyl group typically represented by a benzyl group. If the monovalent hydrocarbon group has 21 or more carbon atoms, the raw materials are not easily available. The monovalent hydrocarbon group denoted by R¹ is preferably an alkyl group having 1 to 14 carbon atoms from the viewpoint of gelling power, synthesis easiness and handleability.

R denotes a saturated or unsaturated monovalent hydrocarbon group having a perfluoroalkyl group and having 2 to 22 carbon atoms, and may be an aliphatic hydrocarbon group, and may have further an aromatic hydrocarbon group. In the case where the hydrocarbon group is a monovalent aliphatic hydrocarbon group, the hydrocarbon group may be a branched one or non-branched one. In the case where the monovalent hydrocarbon group has an aromatic hydrocarbon group, the aromatic hydrocarbon group may or may not have further a substituent. The monovalent hydrocarbon group may have a perfluoroalkyl group in the main chain of the molecule, or in the side chain thereof. Further, the monovalent hydrocarbon group may have one perfluoroalkyl group, or two or more perfluoroalkyl groups. In addition, the perfluoroalkyl group may be a straight-chain one or branched-chain one. The perfluoroalkyl group preferably has 2 to 12 carbon atoms, and more preferably 2 to 6 carbon atoms. In addition, the perfluoroalkyl group is preferably a straight-chain one. A longer perfluoroalkyl group exhibits a higher gelling power, and with a shorter perfluoroalkyl group, the availability of the raw materials and the synthesis become easier.

In the compound (2), Ar denotes a substituted or nonsubstituted divalent aromatic group having 8 to 30 atoms of the nucleus(es). The divalent aromatic group is a cyclic divalent group exhibiting so-called "aromaticity." The divalent aromatic group may be a carbocyclic group or a heterocyclic group. These divalent aromatic groups may be ones substituted with a substituent, or may be nonsubstituted ones, which are not substituted. The substituent of the divalent aromatic group is preferably selected from the viewpoint of easily permitting the introduction of a perfluoroalkyl(oligomethylene)thio group and the introduction of a hydrocarbonoxy group as described later.

The carbocyclic group has 10 to 30 atoms of the nucleus(es), and may be one substituted with a substituent or a nonsubstituted one, which is not substituted. Specific examples thereof include divalent groups having a nucleus(es) typically represented by a biphenylene group, a terphenylene group, a naphthylene group, an anthranylene group, a phenanthrylene group, a pyrenylene group, a chrysenylene group, and a fluoranthenylene group. In addition, the carbocyclic group may have two or more of the divalent groups described above (which may be identical to or different from each other) in the range of 10 to 30 atoms of the nucleus(es).

The heterocyclic group has 8 to 30 atoms of the nucleus(es) and examples thereof include divalent groups having two or more nucleuses (which may be identical to or different from each other) typically represented by a furanylene group, a thiophenylene group, a triazolene group, an oxadiazolene group, a pyridylene group, and a pyrimidylene group in the range of 8 to 30 atoms of the nucleus(es).

Further, Ar may be a group having both of the carbocyclic group and the heterocyclic group described above in the range of 8 to 30 atoms of the nucleus(es). Ar having 8 or more atoms of the nucleus(es) exhibits a high gelling power and raises the versatility of selecting the structure of R and the range of the value of m. In addition, the raw materials of the compound (2) in which Ar has 30 or less atoms of the nucleus(es) are easily available, and the synthesis is also easy.

Among these, from the viewpoint of the gelling power and the synthesis easiness, the divalent aromatic group is preferably a group which is a condensed ring having one or more substituted or nonsubstituted aromatic hydrocarbon rings (more preferably benzene rings), or a group having a plurality of aromatic rings bonded to each other by a single bond(s), one or more of the aromatic rings being an aromatic hydrocarbon ring (more preferably a benzene ring). The divalent aromatic group is further preferably a substituted or nonsubstituted biphenylene group, terphenylene group, naphthylene group, anthranylene group, and phenylene pyridylene group (-Ph-Py-, wherein Ph denotes a benzene ring, and Py denotes a pyridine ring), and most preferably a biphenylene group. In addition, examples of the substituent described above include alkyl groups typically represented by a methyl group and an ethyl group, and a halogen atom.

R¹ denotes a saturated or unsaturated monovalent hydrocarbon group having 1 to 20 carbon atoms, and may be an aliphatic hydrocarbon group, and may further have an aromatic hydrocarbon group. In the case where the hydrocarbon group is a monovalent aliphatic hydrocarbon group, the hydrocarbon group may be branched or non-branched. Further in the case where the monovalent hydrocarbon group has an aromatic hydrocarbon group, the aromatic hydrocarbon group may further have a substituent, or no substituent. However, the monovalent hydrocarbon group, in order that the compound (2) is dissolved in a nonaqueous solvent, which is then gelatinized, is preferably a hydrocarbon group,
capable of dissolving the compound (2) in the nonaqueous solvent, such as an arylalkyl group typically represented by a benzyl group. If the monovalent hydrocarbon group has 21 or more carbon atoms, the raw materials are not easily available. The monovalent hydrocarbon group denoted by R¹ is preferably an alkyl group having 1 to 14 carbon atoms from the viewpoint of the gelling power, synthesis easiness, and handleability.

m denotes a natural number of 2 to 16 and is preferably a natural number of 2 to 12, further preferably a natural number of 2 to 6. By adjusting the range of m in the range described above, a compound (2) exhibits a higher gelling power, and handling ability and becomes one excellent in the easiness of synthesis and the easiness of the raw material availability. From the viewpoint of the gelling power of the compound (2), p denotes an integer of 2 to 4.

Further, from the viewpoint of having excellent gelling power for various types of nonaqueous solvents, and from the viewpoint of being able to exist stably as a gelatinous composition, the sum total of the value of m and the number of carbon atoms in R¹ is preferably 7 to 20, more preferably 8 to 16, and further preferably 10 to 14.

The method for synthesizing the compound (1) and the compound (2) is not particularly limited, and the compound (1) and the compound (2) can be synthesized by any method. For example, it is possible to synthesize them by first preparing a skeleton of an aromatic group, and thereafter reacting both the terminals.with an alkyl chain or the like, or it is possible to first prepare chains of both the terminals and finally synthesize a predetermined aromatic compound.

More specifically, the compounds (1) and (2) can be obtained, for example, by the following synthesis method. First, a thiol compound represented by the following general formula (1a) is sulfidized with a compound represented by the following general formula (1b) in a solvent, such as dry THF, in the presence of a base, such as triethylamine, to obtain a compound represented by the following general formula (1c). Here, in formulas (1a), (1b), and (1c), Ar, m, and p have the same meanings as those in formulas (1) and (2), and X¹ denotes a halogen atom, for example, an iodine atom.

HS-Ar-OH (1a)

CₘF₂ₘ₊₁CₚH₂ₚX¹ (1b)

CₘF₂ₘ₊₁CₚH₂ₚ-S-Ar-OH (1c)

Then, the compound represented by the above general formula (1c) is etherified with a compound represented by the following general formula (1d) in a solvent, such as 3-pentanone, in the presence of an alkali metal compound, such as K₂CO₃, to obtain a compound represented by the following general formula (1e). Here, in formulas (1d) and (1e), Ar, R¹, m, and p have the same meanings as those in formulas (1) and (2), and X² denotes a halogen atom, for example, a bromine atom.

R¹X² (1d)

CₘF₂ₘ₊₁CₚH₂ₚ-S-Ar-O-R¹ (1e)

Then, the compound represented by the above general formula (1e) is oxidized by an oxidant, such as hydrogen peroxide, in the presence of a catalyst, such as acetic acid, to obtain the compound (1) and the compound (2).

As such a synthesis method, for example, a synthesis method described in International Publication No. WO 2009/78268 can be referred to.

In addition, in the case where Ar is a group having a plurality of aromatic rings bonded to each other by a single bond(s), such as a biphenylene group, a terphenylene group, and a phenylene pyridylene group, the compound (1) and the compound (2) can be obtained, for example, by a synthesis method described below. First, a thiol compound represented by the following general formula (1f) is sulfidized with a compound represented by the above general formula (1b) in a solvent, such as dry THF, in the presence of a base, such as triethylamine, to obtain a compound represented by the following general formula (1g). Here, in formulae (1f) and (1g), m and p are each the same as those in formula (2), X³ denotes a halogen atom such as a bromine atom, and Ar² denotes a part of the divalent aromatic hydrocarbon group constituting Ar in the above general formula (1).

HS-Ar²-X³ (1f)

CₘF₂ₘ₊₁CₚH₂ₚ-S-Ar²-X³ (1g)

Then, the compound represented by the above general formula (1g) is oxidized with an oxidizing agent, such as hydrogen peroxide, in the presence of a catalyst, such as acetic acid, to obtain a compound (1 h) shown below. Here, in the formula (1 h), Ar², X³, m, and p are each the same as those in the formula (1g).

CₘF₂ₘ₊₁CₚH₂ₚ-SO₂-Ar²-X³ (1h)

Then, from the compound represented by the above general formula (1 h) and a compound represented by the following general formula (1 i), the compound (1) or the compound (2) is obtained in an aqueous solution of a base, such as K₂CO₃, in the presence of a palladium catalyst through Suzuki-Miyaura coupling. Here, in the formula (1i), R¹ is the same as that in the above general formula (1), and Ar³ denotes a part of the divalent aromatic hydrocarbon group constituting Ar in the above general formula (1), which is different from Ar², and Ar² and Ar³ being bonded by a single bond to form Ar.

R¹-O-Ar³-B(C)H)₂ (1i)

The compound (1) and the,compound (2) can be used as a gelling agent for gelatinizing nonaqueous solvents. Particularly, such compounds are advantageous in that various types of nonaqueous solvents can be gelatinized,or solidified by adding small amounts of the compounds. In addition, a described gelatinous composition contains one or two or more compounds (1) or compounds (2) and a nonaqueous solvent.

The nonaqueous solvent contained in the described gelatinous composition is not particularly limited, but a nonaqueous solvent which is liquid at room temperature is generally used.

Examples of such a nonaqueous solvent include alcohols such as methanol, ethanol, isopropanol, butanol, and octanol, acid esters such as methyl acetate, ethyl acetate, propyl acetate, butyl acetate, γ-butyrolactone, γ-valerolactone, and ε-caprolactone, ketones such as dimethyl ketone, diethyl ketone, methyl ethyl ketone, 3-pentanone, and acetone, hydrocarbons such as pentane, hexane, octane, cyclohexane, benzene, toluene, xylene, fluorobenzene, and hexafluorobenzene, ethers such as diethyl ether, 1,2-dimethoxyethane, 1,4-dioxane, crown ethers, glymes, tetrahydrofuran, and fluoroalkyl ether, amides such as N,N-dimethylacetamide, N,N-dimethylformamide, ethylenediamine, and pyridine, carbonates such as propylene carbonate, ethylene carbonate, vinylene carbonate, fluoroethylene carbonate, diethyl carbonate, and ethyl methyl carbonate, nitriles such as acetonitrile, propionitrile, adiponitrile, and methoxyacetonitrile, lactams such as N-methylpyrrolidone (NMP), sulfones such as sulfolane, sulfoxides such as dimethyl sulfoxide, industrial oils such as silicon oil and petroleum, and edible oil.

In addition, as a nonaqueous solvent, ionic liquids may also be used. The ionic liquids refer to a normal temperature molten salt composed of combined ions of organic cations and anions. The ionic liquids have features of flame retardant, low explosibility, and almost no vapor pressure. In addition, The ionic liquids are expected to be developed to various types of applications because the ionic liquids have a high thermal and ionic conductivities, can be controlled and designed in physical properties by the selection of ion species, and have a selective and high gas absorption power, and the like.

Examples of the organic cation include imidazolium ions such as a dialkylimidazolium cation and a trialkylimidazolium cation, tetraalkylammonium ions, alkylpyridinium ions, dialkylpyrrolidinium ions, and dialkylpiperidinium ions.

Examples of anions usable as counter ions of the organic cations are a PF₆ anion, a PF₃(C₂F₅)₃ anion, a PF₃(CF₃)₃ anion, a BF₄ anion, a BF₂(CF₃)₂ anion, a BF₃(CF₃) anion, a bisoxalatoborate anion, a Tf (trifluoromethanesulfonyl) anion, an Nf (nonafluorobutanesulfonyl) anion, a bis(fluorosulfonyl)imide anion, a bis(trifluoromethanesulfonyl)imide anion, a bis(pentafluoroethanesulfonyl)imide anion, a dicyanoamine anion, a halide anion, and the like.

These nonaqueous solvents are used singly or in combination of two or more.

The described gelatinous composition preferably contains 0.05 to 10.0% by mass, more preferably 0.1 to 5.0% by mass, and further preferably 0.3 to 3.0% by mass, of the compound (1) or the compound (2) based on the total amount of the gelatinous composition. The content equal to or higher than the lower limit value described above provides a tendency that the compound (1) or the compound (2) functions more fully as a gelling agent The content equal to or lower than the upper limit value described above provides a tendency of giving more improved economical efficiency and handling ability, and can further inhibit the gelling agent from becoming an impurity and can further prevent the performances of the nonaqueous solvent from decreasing.

The described gelatinous composition contains preferably 80 to 99.95% by mass of the nonaqueous solvent, more preferably 90 to 99.9% by mass thereof, and further preferably 90 to 99.7% by mass thereof, based on the total amount of the gelatinous composition. The content equal to or higher than the lower limit value described above provides a tendency that the performances of the nonaqueous solvent can further be prevented from decreasing; and the content equal to or lower than the upper limit value described above provides a tendency that the compound (1) or the compound (2) more fully functions as a gelling agent.

The described gelatinous composition may contain, in addition to the compound (1) or the compound (2) and the nonaqueous solvent, other components in the range of not inhibiting the function of the compound (1) or the compound (2) as a gelling agent. Examples of such components include gelling agents other than the compound (1) or the compound (2), coagulants, thickening agents, stabilizers, antioxidants, emulsifiers, lubricants, and a safety-improving additives.

The method for preparing the gelatinous composition is not particularly limited. The gelatinous composition can be prepared, for example, by mixing a nonaqueous solvent, a gelling agent (that is, the compound (1) or the compound (2)), other additives, and the like under heating to prepare a homogeneous mixed liquid, and thereafter decreasing the temperature of the mixed liquid. The mixing order of each component is not particularly mattered, but if a solution composed of a nonaqueous solvent and additives is prepared in advance and thereafter a gelling agent is mixed, these can prepare more easily a homogeneous mixed liquid, which is preferable.

An electrode according to the present embodiment is an electrode for an electrochemical device, containing one or two or more compounds (1) or compounds (2). A method for containing the compound (1) or the compound (2) in the electrode may involve, for example, simultaneously introducing the compound (1) or the compound (2) when an electrode active substance mixture is prepared, or later applying and coating the compound (1) or the compound (2) on a fabricated electrode. The method for applying or coating the compound (1) or the compound (2) on the electrode is not particularly limited, and may involve, for example, preparing a solution or a slurry in which the compound (1) or the compound (2) is dissolved or dispersed in a solvent (preferably a nonaqueous solvent), and applying or coating the solution or the slurry on an electrode by a bar coat method, or applying or coating by a cast method. Alternatively, the solution or the slurry may be applied by a spray method or brush coating.

By containing the compound (1) or the compound (2) in the electrode for an electrochemical device, the safety, reliability, and durability of the electrode and an electrochemical device comprising the electrode are improved.

The content of the compound (1) or the compound (2) in the electrode in the present embodiment is not particularly limited as long as the content is in the range of not inhibiting the function of the electrode. The content is, from the viewpoint of adhesive retention and safety improvement, preferably 0.1 to 20.0 parts by mass, and more preferably 1.0 to 10.0 parts by mass, based on 100 parts by mass of the electrode active substance.

Examples of an electrochemical device in the present embodiment include secondary batteries and storage batteries typically represented by lithium ion secondary batteries, capacitors typically represented by lithium ion capacitors and electric double layer capacitors, and power generation members typically represented by fuel cells and solar batteries. Among these, the electrode in the present embodiment is preferably used in lithium ion secondary batteries and lithium ion capacitors. The electrochemical device in the present embodiment may have a conventionally known structure except that the above electrode is used.

A described electrolyte solution for a dye-sensitized solar battery comprises one or two or more compounds (1) or compounds (2) and preferably further comprises a nonaqueous solvent and an electrolyte. The nonaqueous solvent used in the electrolyte solution for a dye-sensitized solar battery is not particularly limited, and various types thereof can be used. A solvent having a nitrile group such as acetonitrile, propionitrile, or methoxyacetonitrile, is preferred, and acetonitrile is more preferred. In addition, as the nonaqueous solvent for the electrolyte solution for a dye-sensitized solar battery, also an ionic liquid can be used. As the ionic liquid, various types thereof can be selected, ionic liquids having a cation containing an imidazolium group are given attention to (for example, "Function Creation and Applications of Ionic Liquid", published by NTS Inc. in 2004) and are preferable. In addition, the electrolyte is not particularly limited, and may be electrolytes contained in electrolyte solutions of conventional dye-sensitized solar batteries.

The content of the compound (1) or the compound (2) in the described electrolyte solution for a dye-sensitized solar battery is not particularly limited as long as the content is in the range of not inhibiting the function as a dye-sensitized solar battery. The content is, from the viewpoint of the gelling power and the performance as an electrolyte solution, preferably 0.1 to 7.0% by mass, more preferably 0.5 to 5.0 % by mass.

A carbon dioxide-sorbing composition used in the present embodiment is a composition comprising one or two or more compounds (1) or compounds (2) and an ionic liquid. Carbon dioxide separation and recovery techniques using an ionic liquid is given attention to as environmental techniques (for example, "Separation/Recovery and Storage/Isolation Technologies of CO2", published by NTS Inc. in 2009). The carbon dioxide-sorbing composition has a feature of being capable of physically sorbing CO₂ selectively at nearly normal temperature, and capable of separating and recovering CO₂ in a simple operation. Ionic liquids usable are various types thereof, and are not particularly limited. But ions having an imidazolium site or an ammonium site are preferable as cations. Ions containing fluorine are preferable, and bis(trifluoromethanesulfonylimide) ions are more preferable, as anions. An ionic liquid having a property of sorbing carbon dioxide is gelatinized with a gelling agent of the compound (1) or the compound (2) to thereby prepare a carbon dioxide-sorbing composition thereof.

The content of the compound (1) or the compound (2) in the carbon dioxide-sorbing composition used in the present embodiment is not particularly limited as long as the content is in the range of not inhibiting the function as a carbon dioxide sorbing-material. The content is, from the viewpoint of the gelling power, the carbon dioxide absorption capacity and the handling ability, preferably 0.1 to 10.0% by mass, and more preferably 1.0 to 5.0% by mass.

The fluoroalkane derivative used in the present embodiment can gelatinize or solidify relatively many types of nonaqueous solvents in the addition of a small amount thereof, for example, 10% or less, of the fluoroalkane derivative. Moreover, a gelatinous composition using the fluoroalkane derivative hardly transforms to a sol even at a relatively high temperature, and can exist stably over a long period as a gel. Additionally, since the fluoroalkane derivative usually has no hydrogen bondability or a weak one, the function of the gelling agent is secured even in non aqueous solvents and systems in which hydrogen bonds cannot be stably present.

The embodiment to carry out the present invention has been described, but the present invention is not limited to the present embodiment described above.

### Examples

Hereafter, the present invention will be described further in detail by way of Examples, but the present invention is not limited to these Examples. The properties of gelatinous compositions were measured and evaluated as follows.

### (i) Evaluation of Gelling power

A gelling agent and a nonaqueous solvent (an ionic liquid may be used as one type thereof in some cases) were mixed under heating in a vessel to make a homogeneous mixed liquid, which was thereafter cooled to 25°C to obtain a sample liquid. The heating was carried out until the gelling agent was dissolved, and the final temperature was in the range of 70°C to 100°C. The vessel was allowed to stand at an environment of 25°C for 30 min, and thereafter turned upside down in the state of the sample liquid accommodated therein, and the fluidity at this time was observed to evaluate the gelling power. A material which had lost fluidity was regarded as being gelatinized, and evaluated as "a gelatinous composition"; and by changing the mixing ratio of a nonaqueous solvent and a gelling agent, the lowest concentration of the gelling agent necessary for making a gelatinous composition (the concentration of the gelling agent based on the total amount of the gelatinous composition) was determined as a gelling concentration. A smaller amount of a gelling agent can be said to exhibit a higher gelling power. The results are shown in Tables 1 to 3 and 5. In Tables 1 to 3 and 5, "%" means % by mass.

### (ii) Evaluation of stability of a gel

A sample liquid (the concentration of a gelling agent was the lowest one necessary for making a gelatinous composition) containing a nonaqueous solvent, prepared in "(i) Evaluation of the gelling power", was allowed to stand at 25°C for 3 days, visually judged for the gel state, and evaluated as follows. The observation of the fluidity was carried out by turning upside down the vessel accommodating the sample liquid and observing the fluidity at this time. The results are shown in Table 4.
A: a gel was stably present also after still standing.
B: a small amount of a nonaqueous solvent oozed out from the gel after still standing.
C: the fluidity was redeveloped, or a gelling agent and a nonaqueous solvent were phase-separated, after still standing.

### (iii) Lithium deposition test of a lithium ion secondary battery

A lithium deposition test was carried out using a fabricated lithium ion battery being a monolayer laminate type battery equipped with electrodes, described later. The battery charged to 4.2 V at a constant current of 9.0 mA was discharged to 3.0 V, and further charged at a constant current of 45 mA for 1.5 hours. The charged battery was disassembled in an atmosphere of a dew point of -60°C or lower and a moisture concentration of 10 ppm or less. The negative electrode surface of the disassembled battery was observed by an optical microscope of a magnification of 2,000x to evaluate the behavior of lithium deposition based on the following standard.
A: there was recognized no lithium deposition.
B: there was recognized lithium deposition, but the surface of the deposit was smooth.
C: there was recognized lithium deposition, and sharp dendrite was recognized on the surface of the deposit.

The deposition of dendrite causes battery short circuit, and causes a decrease in the safety of the battery.

### (iv) Test of the carbon dioxide absorption capacity

The carbon dioxide absorption capacity was tested at each carbon dioxide pressure by a weight method using a magnetic levitation-type balance (trade name: "MSB-AD", made by BEL Japan, Inc.). The carbon dioxide absorption capacity was evaluated as a carbon dioxide absorption capacity per unit weight of a sample in terms of an amount of carbon dioxide at 25°C and 101.3 kPa (1 atm).

### (Examples 1 to 8 and Comparative Examples 1 to 3)

The compounds represented by the following formulas (3) to (12) were evaluated for the gelling power by adding any one of the nonaqueous solvents shown in Tables 1 to 3. The results are shown in Tables 1 to 3.

The compounds represented by the following formulas (4) to (12) were evaluated for the gel stability by adding any one of the nonaqueous solvents shown in Table 4. The results are shown in Table 4.

### (Examples 9 to 11)

The compounds represented by the following formulas (3), (4), and (13) were evaluated for the gelling power by adding any one of the ionic liquids shown in Table 5. The results are shown in Table 5.

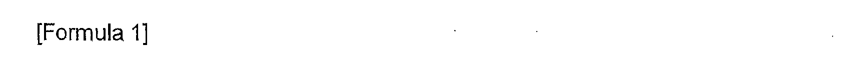

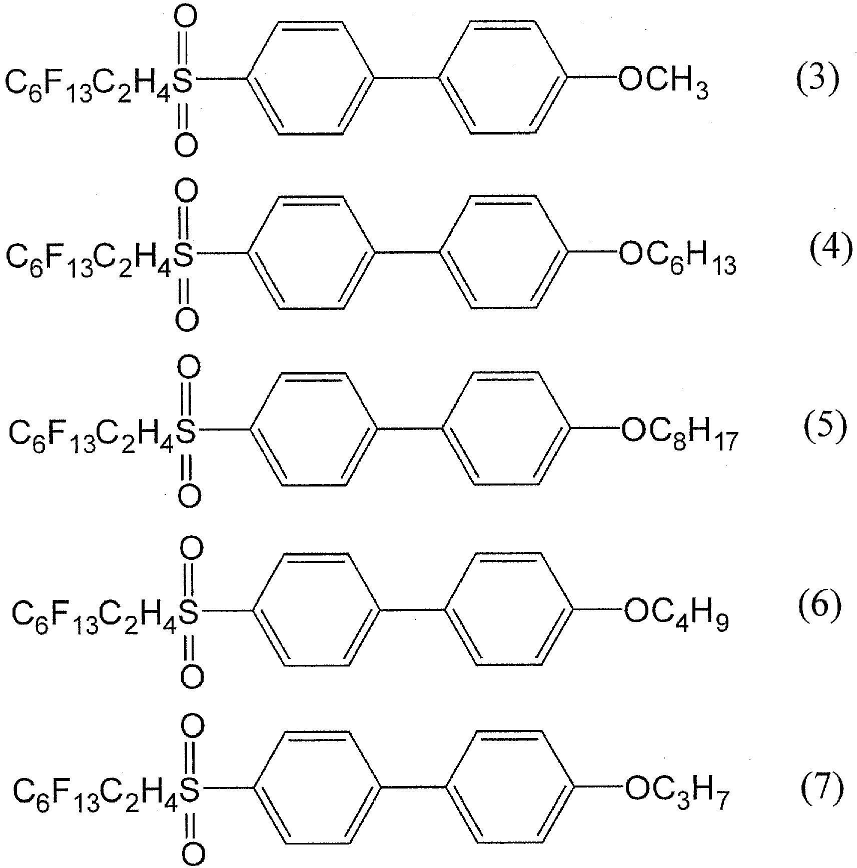

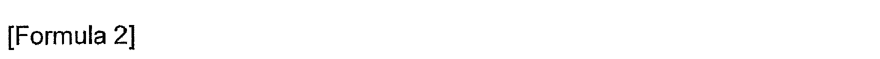

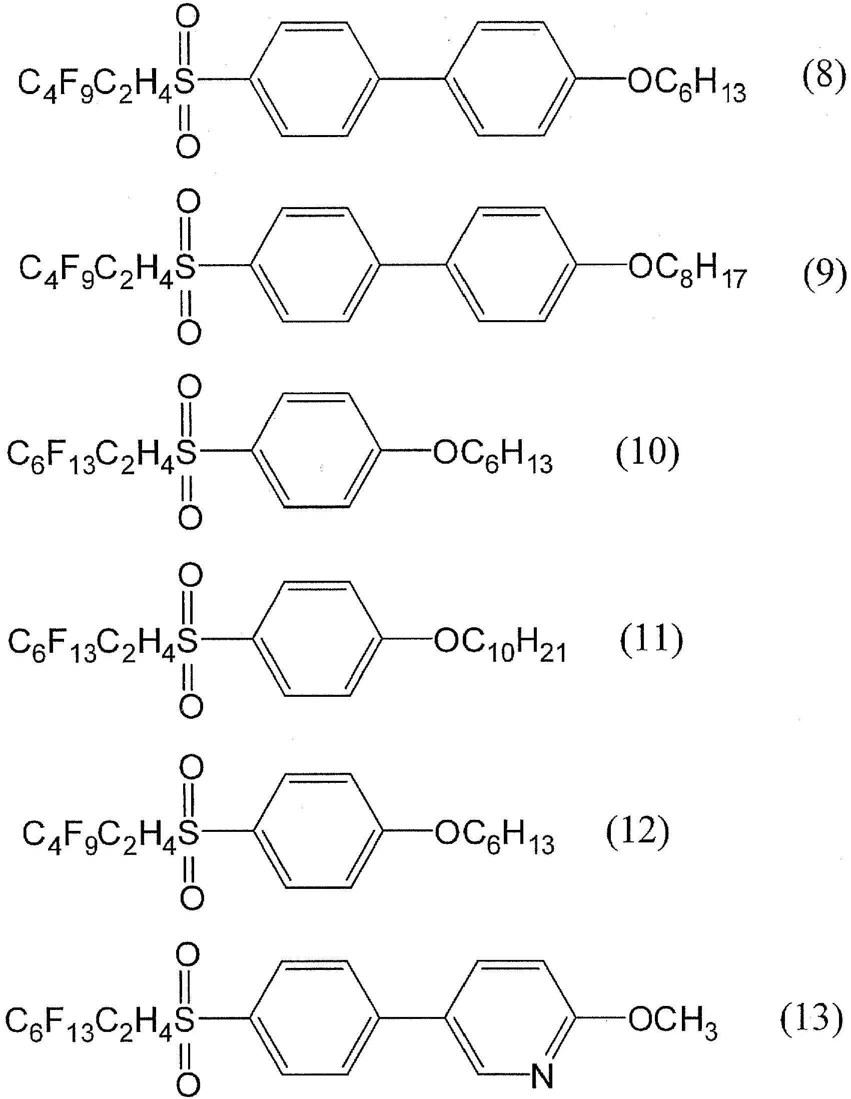

The compound represented by the above formula (3) (referred to as the compound (3); the same applies hereinafter) was synthesized as follows.

A compound (a) was prepared according to the following scheme. Specifically, 4.88 g (4.82 × 10⁻² mol) of triethylamine was added to a 100-mL dry tetrahydrofuran (dry THF) solution of 15.04 g (3.17 × 10⁻² mol) of 2-(perfluorohexyl)ethyl iodide and 5.97 g (3.16 × 10⁻² mol) of p-bromothiophenol in a 200-mL round-bottomed flask, and the solution was refluxed in an oil bath at 84°C for 20 hours. After the temperature of the solution was returned to room temperature, since a solid was observed in the solution, the solid was removed by suction filtration.

After the filtrate was transferred to a 300-mL separating funnel, cyclopentyl methyl ether was added, and an organic phase was washed twice with water, and anhydrous magnesium sulfate was added to the organic phase to dry the organic phase. The anhydrous magnesium sulfate was removed by filtration. An obtained filtrate was concentrated under reduced pressure, and a residue was recrystallized with ethanol. As a result, 12.36 g of a compound (a) was obtained (yield: 73%, melting point: 39 to 41°C). The structure of the compound (a) was identified by ¹H-NMR.

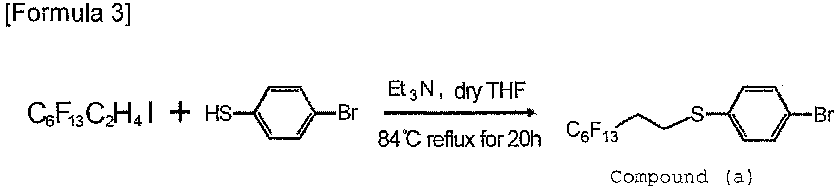

Then, a compound (b) was obtained according to the following scheme. Specifically, 2.75 g (2.83 × 10⁻² mol) of a 35% hydrogen peroxide solution was added to a 50-mL glacial acetic acid solution of 5.01 g (9.36 × 10⁻³ mol) of the compound (a) in a 300-mL round-bottomed flask, and stirred in an oil bath at 70°C for 89 hours. After the temperature of the solution was returned to room temperature, 5 mL of a 20% sodium hydrogensulfite aqueous solution was added to reduce unreacted hydrogen peroxide. At this time, although a solid already deposited in the solution, when 90 mL of water was added, a solid further deposited. After suction filtration, the solid was washed with water. As a result, 4.74 g of a compound (b) was obtained (yield: 89%, melting point: 127 to 129°C). The structure of the compound (b) was identified by ¹H-NMR.

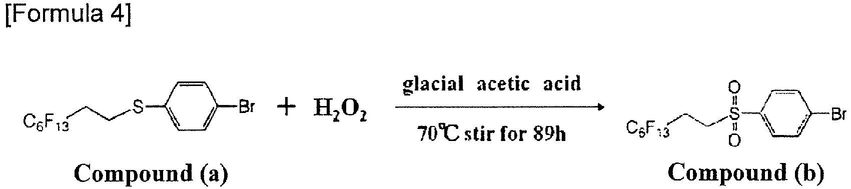

Then, a compound (f), that is, the compound (3), was obtained according to the following scheme. Specifically, 0.60 g (3.95 × 10⁻³ mol) of 4-methoxyphenyl borate, 2.24 g (3.95 × 10⁻³ mol) of the compound (b), 30 mL of a 2M sodium carbonate aqueous solution, and 40 ml of 1,4-dioxane (an amount added until the solid was dissolved) were added in a 100-mL round-bottomed flask. After 0.13 g (5.80 × 10⁻⁴ mol) of palladium diacetate and 0.63 g (2.36 × 10⁻³ mol) of triphenylphosphine were added thereto, a Dimroth tube was attached to the round-bottomed flask, and the mixture was vigorously stirred in a N₂ atmosphere at 95°C for 2.5 hours. After the mixture was cooled to room temperature, 50 mL of water was added to the mixture, which was then stirred at room temperature for 2.5 hours. After the mixture was transferred to a 300-mL separating funnel, 80 mL of ethyl acetate an organic solvent was added, and a water phase was removed. The water phase was extracted twice with 50 mL of ethyl acetate. These organic phases were combined, and washed once with 120 mL of a saturated sodium hydrogencarbonate aqueous solution, and twice with saturated brine. After the organic phase was transferred to a 200-mL conical flask, 0.2 g of active carbon was added to the organic phase, which was then stirred at room temperature for 30 min. Sodium sulfate was further added and stirred at room temperature for 1 hour.

Celite was spread to a depth of 1 cm in a Nutsche, and a solid was removed by suction filtration using the Nutsche. After a filtrate was transferred to a 200-mL round-bottomed flask, the filtrate was concentrated under reduced pressure to obtain a solid (c) (the amount of the solid (c): 2.18 g). 20 mL of petroleum ether and 30 mL of methanol were added to the solid (c), but since the solid was not completely dissolved, the mixture was suction-filtrated to make a filtrate and a solid (d) (the amount of the solid (d): 1.24 g). The filtrate was concentrated under reduced pressure to make a solid (e) (the amount of the solid (e): 0.78 g). As a result of the measurement of ¹H-NMR spectra of the solids (d) and (e), the main component of the solid (d) was found to be a target material. Since black microcrystals were observed in the solution when the solid (d) was dissolved in chloroform, the solution was taken by a glass injection syringe, and the microcrystals were removed through a cylinder filter (pore diameter: 0.45 µm, diameter: 13 mm). The solution passed through the filter was concentrated under reduced pressure, and the recrystallization was carried out with chloroform. In order to raise the yield, the filtrate after the recrystallization was concentrated under reduced pressure, and the recrystallization was twice carried out with chloroform and with ethanol. As a result of the recrystallizations of 3 times in total, 0.87 g of a compound (f) was obtained (yield: 37%, melting point: 187 to 189°C). The structure of the obtained compound (f) was identified by ¹H-NMR (CDCl₃) and ¹⁹F-NMR (CDCl₃). The results were as follows.
¹H-NMR(CDCl₃) 2.63 (2H, m), 3.36 (2H, m), 3.88 (3H, s), 7.03 (2H, d, J=8.5 Hz), 7.58 (2H, d, J=8.5 Hz), 7.77 (2H, d, J=8.5 Hz) 7.97 (2H, d, J=8.5 Hz) ppm ¹⁹F-NMR(CDCl₃) -126.60 (2F, m), -123.60 (2F, m), -123.34 (2F, m), -122.35 (2F, m), - 114.02 (2F, m), -81.25 (3F, m) ppm

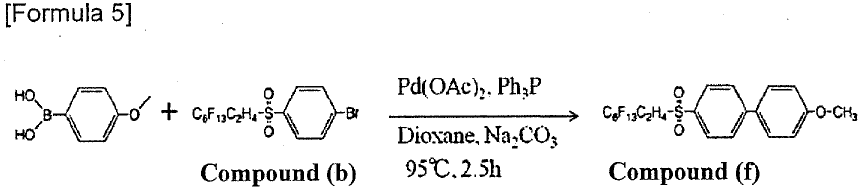

In addition, the compound represented by the above formula (4) (referred to as the compound (4); the same applies hereinafter) was synthesized as follows. The compound (5), the compound (6), the compound (7), the compound (8), and the compound (9) were also synthesized according to the synthesis method of the compound (4).

First, a compound (a) was obtained according to the following scheme. Specifically, 11.34 g (60 mmol) of p-bromothiophenol was charged in a 200-mL round-bottomed flask in a nitrogen atmosphere, and 70 mL of DME was added thereto. 29.86 g (63 mmol) of 2-(perfluorohexyl)ethyl iodide and 12.42 g (90 mmol) of K₂CO₃ were further charged, and the mixture was heated at 50°C and stirred for 3 hours. After the temperature of the mixture was returned to room temperature, a solid remaining in the mixture was removed by suction filtration. A filtrate after the removal of the solid was concentrated under reduced pressure. A high-viscosity oily material was obtained by the concentration, and then, the oily material was vacuum-dried at 50°C, and the remaining solvent and unreacted materials were distilled out. As a result, 32.82 g of a compound (a) was obtained. The structure of the compound (a) was identified by ¹H-NMR (CDCl₃).

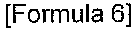

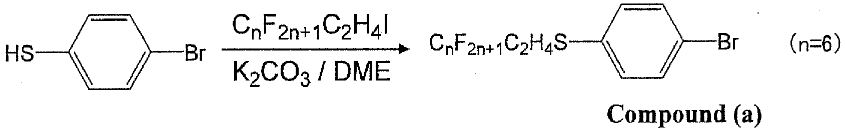

Then, a compound (b) was obtained according to the following scheme. Specifically, 26 mL (300 mmol) of a 35% hydrogen peroxide solution was added to a 100-mL glacial acetic acid solution of 32.82 g of the compound (a) in a 200-mL round-bottomed flask under a nitrogen atmosphere, and the mixture was stirred in an oil bath at 70°C for 2 hours. After water was added thereto, a white solid produced was filtered by suction filtration; water was added to wash the solid twice; and hexane was further added to once wash the solid. The solid was further dried under reduced pressure at 90°C to obtain 26.34 g of a compound (b) (yield: 75%). The structure of the compound (b) was identified by ¹H-NMR (CDCl₃).

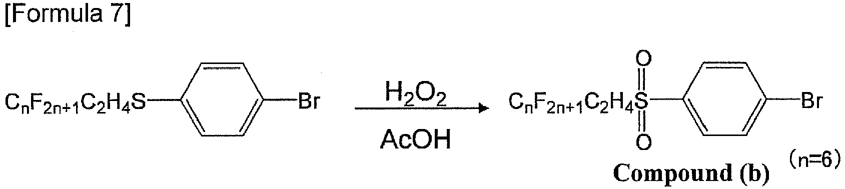

Then, a compound (j) was obtained according to the following scheme. Specifically, 4.4 g (20 mmol) of a compound (i) shown below and 70 mL of 3-pentanone were charged in a 200-mL round-bottomed flask in a nitrogen atmosphere, and stirred at room temperature, and thereafter 4.13 g (25 mmol) of C₆H₁₃Br and 4.14 g (30 mmol) of K₂CO₃ were further added thereto, and refluxed in an oil bath at 120°C for 11 hours. After the temperature of the mixture was returned to room temperature, a solid remaining therein was removed by suction filtration. A filtrate after the removal of the solid was concentrated under reduced pressure to thereby obtain a brown oily material, and then, the oily material was vacuum-dried at 80°C to quantitatively obtain 6.87 g of a solid compound (j). The structure of the compound (j) was identified by ¹H-NMR (CDCl₃).

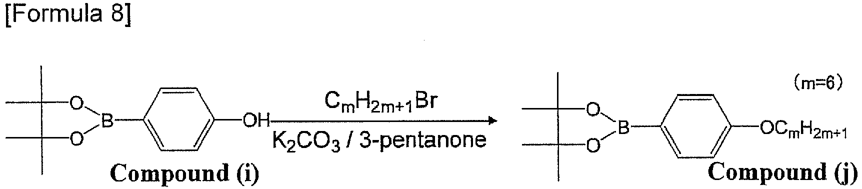

Then, a compound (k), that is, the compound (4), was obtained according to the following scheme. Specifically, 2.0 g (6.58 mmol) of the compound (j), 3.7 g (6.58 mmol) of the compound (b), 60 ml of 1,4-dioxane, 0.295 g (1.31 mmol) of palladium diacetate, 1.18 g (4.5 mmol) of triphenylphosphine, and a 2M sodium carbonate aqueous solution (in which 7 g of sodium carbonate was dissolved in 30 mL of water) were charged in a 200-mL round-bottomed flask. Then, a Dimroth tube was attached to the round-bottomed flask, and the mixture was heated and held at 95°C for 120 min in a nitrogen atmosphere. Thereafter, the mixture was cooled to room temperature, and the separation into an upper phase (gelatinous) and a lower phase (liquid) was made sure of; and ethanol was added thereto in order to azeotropically boil water, and the solvent was thereafter distilled out by an evaporator. After ethyl acetate was added to the round-bottomed flask after the solvent had been distilled out, and the content was heated at 70°C to be dissolved, the ethyl acetate was filtered under the heating state. Then, a vessel containing an obtained filtrate was cooled to room temperature for the filtrate to be again gelatinized. Then, the obtained gelatinous material was washed three times with hexane until the supernatant solution became transparent. A solid obtained by filtering the washed gelatinous material was dried at 70°C under reduced pressure to obtain 2.38 g of a compound (k) (a white solid).

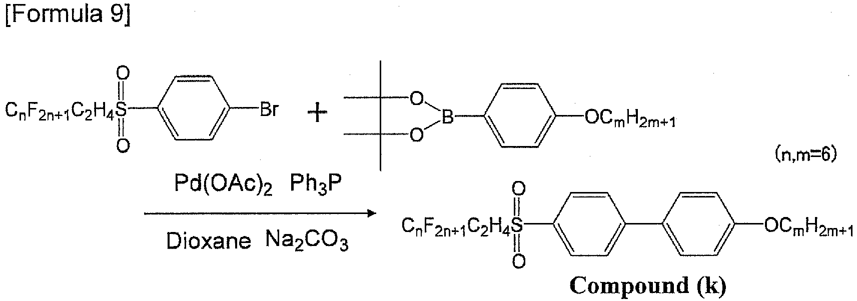

The structure of the obtained compound (k), that is, the compound (4), was identified by ¹H-NMR (CDCl₃) and ¹⁹F-NMR (CDCl₃). The results were as follows. ¹H-NMR(CDCl₃) 0.92 (3H, m), 1.36 (6H, m), 1.81 (2H, m), 2.65 (2H, m), 3.35 (2H, m), 4.02 (2H, m), 7.01 (2H, d, J=8.0 Hz), 7.56 (2H, d, J=8.0 Hz), 7.77 (2H, d, J=8.0Hz), 7.95 (2H, d, J=8.0 Hz) ppm
¹⁹F-NMR(CDCl₃) -126.62 (2F, m), -123.60 (2F, m), -123.35 (2F, m), -122.36 (2F, m),-114.01 (2F, m), -81.25 (3F, m) ppm

The structures of the compound (5), the compound (6), the compound (7), the compound (8), and the compound (9), also were similarly identified by ¹H-NMR (CDCl₃) and ¹⁹F-NMR (CDCl₃). The results were as follows.

### [Compound (5)]

¹H-NMR(CDCl₃) 0.90 (3H, m), 1.31 (10H, m), 1.83 (2H, m), 2.67 (2H, m), 3.37 (2H, m), 4.03 (2H, m), 7.01 (2H, d, J=8.0 Hz), 7.58 (2H, d, J=8.0 Hz), 7.78 (2H, d, J=8.0 Hz), 7.97 (2H, d, J=8.0 Hz) ppm
¹⁹F-NMR(CDCl₃) -126.62 (2F, m), -123.60 (2F, m), -123.34 (2F, m), -122.36 (2F, m), - 114.01 (2F, m), -81.25 (3F, m) ppm

### [Compound (6)]

¹H-NMR(CDCl₃) 0.91 (3H, m), 1.42 (2H, m), 1.81 (2H, m), 2.65 (2H, m), 3.35 (2H, m), 4.03 (2H,m), 7.01 (2H, d, J=8.0 Hz), 7.57 (2H, d, J=8.0 Hz), 7.78 (2H, d, J=8.0 Hz), 7.97 (2H, d, J=8.0 Hz) ppm
¹⁹F-NMR(CDCl₃) -126.62 (2F, m), -123.60 (2F, m), -123.34 (2F, m), -122.37 (2F, m), - 114.02 (2F, m), -81.25 (3F, m) ppm

### [Compound (7)]

¹H-NMR(CDCl₃) 0.90 (3H, m), 1.82 (2H, m), 2.65 (2H, m), 3.34 (2H, m), 4.02 (2H,m), 7.00 (2H, d, J=8.0 Hz), 7.56 (2H, d, J=8.0 Hz), 7.76 (2H, d, J=8.0 Hz), 7.96 (2H, d, J=8.0 Hz) ppm
¹⁹F-NMR(CDCl₃) -126.62 (2F, m), -123.60 (2F, m), -123.34 (2F, m), -122.36 (2F, m), - 114.02 (2F, m), -81.25 (3F, m) ppm

### [Compound (8)]

¹H-NMR(CDCl₃) 0.92 (3H, m), 1.35 (6H, m), 1.80 (2H, m), 2.63 (2H, m), 3.35 (2H, m), 4.02 (2H, m), 7.01 (2H, d, J=8.0 Hz), 7.56 (2H, d, J=8.0 Hz), 7.77 (2H, d, J=8.0 Hz), 7.95 (2H, d, J=8.0 Hz) ppm
¹⁹F-NMR(CDCl₃) -126.62 (2F, m), -124.50 (2F, m), -114.21 (2F, m), -81.45 (3F, m) ppm

### [Compound (9)]

¹H-NMR(CDCl₃) 0.89 (3H, m), 1.35 (10H, m), 1.81 (2H, m), 2.65 (2H, m), 3.35 (2H, m), 4.01 (2H, m), 7.01 (2H, d, J=8.0 Hz), 7.56 (2H, d, J=8.0 Hz), 7.77 (2H, d, J=8.0 Hz), 7.95 (2H, d, J=8.0 Hz) ppm
¹⁹F-NMR(CDCl₃) -126.50 (2F, m), -124.56 (2F, m), -124.24 (2F, m), -81.46 (3F, m) ppm

In addition, the compound (10) and the compounds (11) and (12) were synthesized according to the synthesis methods described in WO 2009/78268. The structures of the compound (10), the compound (11), and the compound (13) were identified by ¹H-NMR (CDCl₃) and ¹⁹F-NMR (CDCl₃). The results were as follows.

### [Compound (10)]

¹H-NMR(CDCl₃) 0.91 (3H, m), 1.36 (6H, m), 1.82 (2H, m), 2.57 (2H, m), 3.29 (2H, m), 4.04 (2H, m), 7.04 (2H, d, J=8.0 Hz), 7.83 (2H, d, J=8.0 Hz) ppm ¹⁹F-NMR(CDCl₃) -126.59 (2F, m), -123.61 (2F, m), -123.32 (2F, m), -122.35 (2F, m), - 114.00 (2F, m), -81.26 (3F, m) ppm

### [Compound (11)]

¹H-NMR(CDCl₃) 0.88 (3H, m), 1.27 (14H, m), 1.81 (2H, m), 2.57 (2H, m), 3.29 (2H, m), 4.04 (2H, m), 7.03 (2H, d, J=8.0 Hz), 7.83 (2H, d, J=8.0 Hz) ppm ¹⁹F-NMR(CDCl₃) -126.61 (2F, m), -123.63 (2F, m), -123.33 (2F, m), -122.36 (2F, m), - 114.00 (2F, m), -81.24 (3F, m) ppm

### [Compound (12)]

¹H-NMR(CDCl₃) 0.91 (3H, m), 1.35 (6H, m), 1.81 (2H, m), 2.57 (2H, m), 3.29 (2H, m), 4.04 (2H, m), 7.03 (2H, d, J=12.0 Hz), 7.83 (2H, d, J=8.0 Hz) ppm ¹⁹F-NMR(CDCl₃) -126.49 (2F, m), -124.59 (2F, m), -114.24 (2F, m), -81.48 (3F, m) ppm

The compound (13) was synthesized as in the following scheme. Specifically, 1.21 g (0.0021 mol, the compound (b)) of 1-bromo-4-[(3,3,4,4,5,5,6,6,7,7,8,8,8-tridecafluorooctyl)sulfonyl]benzene, 0.5 g (0.0021 mol) of 2-methoxy-5-(4,4,5,5-tetramethyl-1,3,2-dioxaborolan-2-yl)pyridine, 40 mL of a 2M sodium carbonate aqueous solution, and 40 mL of 1,4-dioxane were added to a 100-mL round-bottomed flask in a nitrogen atmosphere. Triphenylphosphine (0.34 g, 0.0013 mol) and palladium acetate (0.0754 g, 0.00034 mol) were further added thereto, and the mixture was vigorously stirred at 95°C for 2.5 hours. After the flask atmosphere was returned to the air atmosphere, 50 mL of water was added thereto at room temperature, and stirred for 30 min to cool the interior.

After the completion of the reaction, a solid was observed in the flask, and ethyl acetate was added to dissolve the solid. The content was transferred to a separating funnel; after a water phase was removed, an organic phase was washed three times with 1 M hydrochloric acid, and washed once with water and with brine each. Magnesium sulfate was added thereto to dry the organic phase, and magnesium sulfate was removed by filtration. A filtrate was concentrated by an evaporator, and purified by silica gel column chromatography; and the recrystallization was carried out with ethanol to obtain 0.64 g of a solid (yield: 51%).

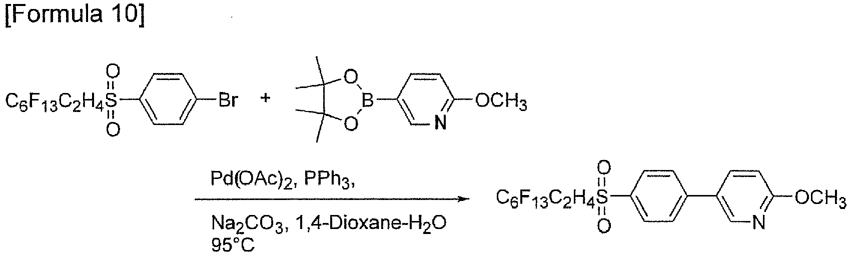

The structure of the obtained compound (13) was identified by IR (KBr) and ¹H-NMR (CDCl₃). The results were as follows.
IR (KBr) v=1138.0, 1151.5, 1197.8, 1209.4, 1234.4, 1294.2, 1485.2, 1606.7 cm^{-1 1}H-NMR (CDCl₃) 2.24 (2H, m), 3.36 (2H, m), 4.01 (3H, s), 6.88 (1H, d, J=8.5 Hz), 7.76 (2H, d, J=8.5 Hz), 7.83 (1H, dd, J=8.5 Hz, 2.5 Hz), 8.00 (2H,d, J=8.5 Hz), 8.45 (1 H, d, J=2.5 Hz) ppm

**[Table 1]**

| | | Example 1 |
|---|---|---|
| Gelling agent type | | (3) |
| Nonaqueous solvents | Ethanol | 5% |
| | Propylene carbonate | 0.8% |
| | γ-butyrolactone | 1.0% |

**[Table 2]**

| | | Example 2 |
|---|---|---|
| Gelling agent type | | (4) |
| Nonaqueous solvents | Ethyl methyl carbonate | 0.8% |
| | Propylene carbonate | 0.5% |
| | γ-butyrolactone | 0.5% |
| | Acetonitrile | 0.5% |
| | N-methylpyrrolidone | 0.5% |
| | 3-Pentanone | 0.5% |
| | N,N-dimethylacetamide | 0.6% |
| | Ethyl acetate | 0.6% |
| | 1,4-Dioxane | 0.5% |
| | Tetrahydrofuran | 0.5% |
| | Ethanol | 1.0% |

**[Table 3]**

| | | Ex. 3 | Ex. 4 | Ex. 5 | Ex. 6 | Ex. 7 | Ex. 8 | Comp. Ex. 1 | Comp. Ex. 2 | Comp. Ex. 3 |
|---|---|---|---|---|---|---|---|---|---|---|
| | | Gelling agent type | | | | | | | | |
| | | (4) | (5) | (6) | (7) | (8) | (9) | (10) | (11) | (12) |
| Nonaqueous solvents | Ethanol | 1% | 1% | 1% | 1.5% | 3% | 1% | 5% | 3% | 5% |
| | Acetonitrile | 0.5% | 1% | 1% | 1% | 1% | 1% | Not gelatinzed | 3% | Not gelatinized |
| | γ-butyrolactone | 0.5% | 1% | 0.7% | 1% | 0.7% | 0.7% | 3% | 3% | Not gelatinized |
| | NMP | 0.5% | 0.5% | 0.5% | 1.5% | 1.0 % | 1.0% | Not gelatinized | 3% | Not gelatinized |

**[Table 4]**

| | | Ex. 3 | Ex. 4 | Ex. 5 | Ex. 6 | Ex. 7 | Ex. 8 | Comp. Ex. 1 | Comp. Ex. 2 | Comp. Ex. 3 |
|---|---|---|---|---|---|---|---|---|---|---|
| | | Gelling agent type | | | | | | | | |
| | | (4) | (5) | (6) | (7) | (8) | (9) | (10) | (11) | (12) |
| Nonaqueous solvents | Ethanol | A | A | A | B | B | A | C | C | C |
| | Acetonitrile | A | A | A | A | A | A | C | A | C |
| | γ-butyrolactone | A | A | A | A | A | A | A | A | C |
| | NMP | A | A | A | A | A | A | C | A | C |

**[Table 5]**

| | | Example 9 | Example 10 | Example 11 |
|---|---|---|---|---|
| Gelling agent type | | (3) | (4) | (13) |
| Ionic liquids | Bmim TFSI | 1.2% | 0.9% | 1.7% |
| | Emim TFSI | 1.2% | 0.7% | 1.8% |
| | P13 TFSI | 1.3% | 0.8% | 1.8% |
| | TMPA TFSI | 1.9% | 1.0% | 1.7% |
| | DEME TFSI | 1.3% | 0.9% | 1.6% |

| | | | | |
|---|---|---|---|---|
| Bmim TFSI=1-butyl-3-methyl-imidazolium bis(trifluoromethanesulfonyl)imide Emim TFSI=1-ethyl-3-methyl-imidazolium bis(trifluoromethanesulfonyl)imide P13 TFSI= N-methyl-N-propylpyrrolidinium ammonium bis(trifluoromethanesulfonyl)imide TMPA TFSI= N,N,N-trimethyl-N-propylammonium bis(trifluoromethanesulfonyl)imide DEME TFSI= N,N-diethyl-N-(2-methoxyethyl)ammonium bis(trifluoromethanesulfonyl)imide | | | | |

### (Example 12)

### <Fabrication of a positive electrode for a lithium ion battery>

Lithium cobaltate (LiCoO₂) as a positive electrode active substance, acetylene black as an electroconductive aid, and a polyvinylidene difluoride (PVdF) as a binder were mixed in a mass ratio of 89.5 : 4.5 : 6.0. The obtained mixture was further mixed with N-methyl-2-pyrrolidone to prepare a slurry solution. The slurry solution was applied on an aluminum foil of 20 µm in thickness and 200 mm in width; after the solvent was dried and removed, the applied slurry was rolled by a roll press, and further vacuum-dried at 150°C for 10 hours, and punched out into a rectangular shape of 50 mm × 30 mm to obtain a positive electrode. The slurry solution was prepared while with respect to the mixture of the obtained electrode after the vacuum-drying, the solvent amount was regulated so that: the weight basis for one surface became 24.8 g/cm² ± 3%; the thickness for the one surface, 82.6 µm ± 3%; the density, 3.0 g/cm³ ± 3%; and the coating width, 150 mm to the width of 200 mm of the aluminum foil.

### <Fabrication of a negative electrode>

### <Fabrication of a negative electrode>

A graphite carbon powder (trade name: "MCMB25-28", made by Osaka Gas Chemical Co., Ltd.) as a negative electrode active substance, acetylene black as an electroconductive aid, and a polyvinylidene difluoride (PVdF) as a binder were mixed in a mass ratio of 93.0 : 2.0 : 5.0. The obtained mixture was further mixed with N-methyl-2-pyrrolidone to prepare a slurry solution. The slurry solution was applied on an aluminum foil of 14 µm in thickness and 200 mm in width; after the solvent was dried and removed, the applied slurry was rolled by a roll press, and further vacuum-dried at 150°C for 10 hours, and punched out into 52 mm x 32 mm to obtain a negative electrode. The slurry solution was prepared while with respect to the mixture of the obtained electrode after the vacuum-drying, the solvent amount was regulated so that: the weight basis for one surface became 11.8 g/cm² ± 3%; the thickness for the one surface, 84.6 µm ± 3%; the density, 1.4 g/cm³ ± 3%; and the coating width, 150 mm to the width of 200 mm of the aluminum foil.

### <Application of a perfluorocarbon derivative>

5 parts by mass of the compound (4) was mixed in 100 parts by mass of dimethyl carbonate, and dissolved therein at 85°C to obtain a dimethyl carbonate solution (α). The solution (α) was applied on the positive electrode under heating so that the positive electrode active substance and the compound (4) became 4 : 1 in mass ratio. After the application, the dimethyl carbonate was distilled out by continuing heating from the rear surface of the electrode, to cast-coat the compound (4) on the positive electrode.

Similarly, the solution (α) was applied on the negative electrode with the solution (α) in a heated state so that the negative electrode substance and the compound (4) became 2 : 1 in mass ratio; and the dimethyl carbonate was distilled out by further continuing heating to cast-coat the compound (4) on the negative electrode.

### <Assembling of a battery>

Two sheets of laminate film (no drawing processing, thickness: 120 µm, 68 mm × 48 mm) obtained by laminating an aluminum layer and a resin layer were overlapped with the aluminum layers outward, and three sides were sealed to fabricate a laminate cell outer package. Then, a polyethylene microporous membrane (membrane thickness: 20 µm, 53 mm × 33 mm) as a separator was prepared; and a laminate obtained by stacking alternately the positive electrode and the negative electrode coated with the compound (4) as described above through the separator in plural numbers was arranged in the laminate cell outer package. Then, an electrolyte solution was injected in the cell outer package to immerse the laminate in the electrolyte solution. The electrolyte solution used was one in which 1 M of LiPF₆ was dissolved in a mixed solution of ethylene carbonate and methyl ethyl carbonate of 1 : 2 in volume ratio. The injection of the electrolyte solution was carried out by repeating reducing the pressure to 13.3 kPa (100 mmHg) and increasing the pressure to the atmospheric pressure until the bubble generation vanishes. Under a reduced pressure environment of 13.3 kPa (100 mmHg), the remaining one side of the laminate cell outer package was sealed to thereby obtain a lithium ion secondary battery.

For the obtained lithium ion secondary battery, "(iii) Test of lithium deposition of a lithium ion secondary battery" was carried out. The result is shown in Table 6.

### (Examples 12 to 14 and Comparative Example 4)

Batteries were assembled as in Example 12, except for altering the presence and absence of coating of the compound (4) on the each electrode, and "(iii) Test of lithium deposition of a lithium ion secondary battery" was carried out. The evaluation results are shown in Table 6.

**[Table 6]**

| | | Example 12 | Example 13 | Example 14 | Comparative Example4 |
|---|---|---|---|---|---|
| Coating of compound (4) | Positive electrode | present | present | absent | absent |
| | Negative electrode | present | absent | present | absent |
| Deposition | | B | B | B | C |

### (Example 15)

5.3 parts by mass of the compound (13) was added to 100 parts by mass of P13 TFSI being an ionic liquid, and heated to 80°C to dissolve the mixture, and the solution was cooled to room temperature to prepare an evaluation sample. The sample was subjected to "(iv) Test of the carbon dioxide absorption capacity". The evaluation result is shown in Table 7.

**[Table 7]**

| Example 15 | |
|---|---|
| Pressure of Carbon dioxide (MPaG) | sorbed amount of carbon dioxide (mL/g) |
| 0.2 | 3.5 |
| 0.4 | 6.9 |
| 0.6 | 10.5 |
| 0.8 | 13.8 |

### Industrial Applicability

The fluoroalkane derivative, the gelling agent and the gelatinous composition according to the present invention can be used to solidify liquid substances in various types of industrial fields (for example, coating, cosmetic, pharmaceutical and medical, spilled petroleum-treating, electronic and optical, and environmental fields).

## Claims

1. An electrode for an electrochemical device, comprising a fluoroalkane derivative represented by the following general formula (1):
R-SO₂-Ar-O-R¹ (1)
wherein Ar denotes a substituted or nonsubstituted divalent aromatic group having 8 to 30 atoms of the nucleus(es), R¹ denotes a saturated or unsaturated monovalent hydrocarbon group having 1 to 20 carbon atoms, and R denotes a saturated or unsaturated monovalent hydrocarbon group having a perfluoroalkyl group and having 2 to 22 carbon atoms, wherein the R is a group represented by the following general formula (2):
CₘF₂ₘ₊₁CₚH₂ₚ- (2)
wherein m denotes a natural number of 2 to 16, and p denotes an integer of 2 to 4.

2. The electrode for an electrochemical device according to claim 1, wherein the Ar is a condensed ring having one or more aromatic hydrocarbon rings, or a group having a plurality of aromatic rings bonded to each other by a single bond(s), one or more of the aromatic rings being an aromatic hydrocarbon ring.

3. The electrode for an electrochemical device according to claim 1 or 2, wherein the Ar is a biphenylene group.

4. Use of a composition for sorbing carbon dioxide, wherein the composition comprises a fluoroalkane derivative represented by the following general formula (1):
R-SO₂-Ar-O-R¹ (1)
wherein Ar denotes a substituted or nonsubstituted divalent aromatic group having 8 to 30 atoms of the nucleus(es), R¹ denotes a saturated or unsaturated monovalent hydrocarbon group having 1 to 20 carbon atoms, and R denotes a saturated or unsaturated monovalent hydrocarbon group having a perfluoroalkyl group and having 2 to 22 carbon atoms, wherein the R is a group represented by the following general formula (2):
CₘF₂ₘ₊₁CₚH₂ₚ- (2)
wherein m denotes a natural number of 2 to 16, and p denotes an integer of 2 to 4, and an ionic liquid.

5. The use according to claim 4, wherein the Ar is a condensed ring having one or more aromatic hydrocarbon rings, or a group having a plurality of aromatic rings bonded to each other by a single bond(s), one or more of the aromatic rings being an aromatic hydrocarbon ring.

6. The use according to claim 4 or 5, wherein the Ar is a biphenylene group.

## Patentansprüche

1. Elektrode für eine elektrochemische Vorrichtung, umfassend ein Fluoralkanderivat, das durch die folgende allgemeine Formel (1) wiedergegeben wird:
R-SO₂-Ar-O-R¹ (1)
worin Ar für eine substituierte oder unsubstituierte zweiwertige aromatische Gruppe mit 8 bis 30 Atomen des Kerns bzw. der Kerne steht; R¹ für eine gesättigte oder ungesättigte einwertige Kohlenwasserstoffgruppe mit 1 bis 20 Kohlenstoffatomen steht und R für eine gesättigte oder ungesättigte einwertige Kohlenwasserstoffgruppe mit einer Perfluoralkylgruppe und 2 bis 22 Kohlenstoffatomen steht, wobei R eine Gruppe ist, die durch die folgende allgemeine Formel (2) wiedergegeben wird:
CₘF₂ₘ₊₁CₚH₂ₚ- (2)
wobei m für eine natürliche Zahl von 2 bis 16 steht und p für eine ganze Zahl von 2 bis 4 steht.

2. Elektrode für eine elektrochemische Vorrichtung nach Anspruch 1, wobei das Ar ein kondensierter Ring mit einem oder mehreren aromatischen Kohlenwasserstoffringen oder eine Gruppe mit mehreren aromatischen Ringen, die über eine Einfachbindung bzw. Einfachbindungen aneinander gebunden sind, wobei es sich bei einem oder mehreren der aromatischen Ringe um einen aromatischen Kohlenwasserstoffring handelt.

3. Elektrode für eine elektrochemische Vorrichtung nach Anspruch 1 oder 2, wobei das Ar eine Biphenylengruppe ist.

4. Verwendung einer Zusammensetzung zur Sorption von Kohlendioxid, wobei die Zusammensetzung ein Fluoralkanderivat, das durch die folgende allgemeine Formel (1) wiedergegeben wird:
R-SO₂-Ar-O-R¹ (1)
worin Ar für eine substituierte oder unsubstituierte zweiwertige aromatische Gruppe mit 8 bis 30 Atomen des Kerns bzw. der Kerne steht; R¹ für eine gesättigte oder ungesättigte einwertige Kohlenwasserstoffgruppe mit 1 bis 20 Kohlenstoffatomen steht und R für eine gesättigte oder ungesättigte einwertige Kohlenwasserstoffgruppe mit einer Perfluoralkylgruppe und 2 bis 22 Kohlenstoffatomen steht, wobei R eine Gruppe ist, die durch die folgende allgemeine Formel (2) wiedergegeben wird:
CₘF₂ₘ₊₁CₚH₂ₚ- (2)
wobei m für eine natürliche Zahl von 2 bis 16 steht und p für eine ganze Zahl von 2 bis 4 steht, und eine ionische Flüssigkeit umfasst.

5. Verwendung nach Anspruch 4, wobei das Ar ein kondensierter Ring mit einem oder mehreren aromatischen Kohlenwasserstoffringen oder eine Gruppe mit mehreren aromatischen Ringen, die über eine Einfachbindung bzw. Einfachbindungen aneinander gebunden sind, wobei es sich bei einem oder mehreren der aromatischen Ringe um einen aromatischen Kohlenwasserstoffring handelt.

6. Verwendung nach Anspruch 4 oder 5, wobei das Ar eine Biphenylengruppe ist.

## Revendications

1. Électrode pour un dispositif électrochimique, comprenant un dérivé de fluoroalcane représenté par la formule générale (1) suivante :
R-SO₂-Ar-O-R¹ (1)
dans laquelle Ar représente un groupe aromatique divalent substitué ou non substitué ayant 8 à 30 atomes du/des noyau(x), R¹ représente un groupe hydrocarboné monovalent saturé ou insaturé ayant 1 à 20 atomes de carbone, et R représente un groupe hydrocarboné monovalent saturé ou insaturé ayant un groupe perfluoroalkyle et ayant 2 à 22 atomes de carbone, R étant un groupe représenté par la formule générale (2) suivante :
CₘF₂ₘ₊₁CₚH₂ₚ- (2)
dans laquelle m représente un nombre naturel de 2 à 16, et p représente un entier de 2 à 4.

2. Électrode pour un dispositif électrochimique selon la revendication 1, dans laquelle Ar est un cycle condensé ayant un ou plusieurs cycles aromatiques hydrocarbonés, ou un groupe ayant une pluralité de cycles aromatiques liés les uns aux autres par une/des simple(s) liaison(s), un ou plusieurs des cycles aromatiques étant un cycle aromatique hydrocarboné.

3. Électrode pour un dispositif électrochimique selon la revendication 1 ou 2, dans laquelle Ar est un groupe biphénylène.

4. Utilisation d'une composition de sorption du dioxyde de carbone, la composition comprenant un dérivé de fluoroalcane représenté par la formule générale (1) suivante :
R-SO₂-Ar-O-R¹ (1)
dans laquelle Ar représente un groupe aromatique divalent substitué ou non substitué ayant 8 à 30 atomes du/des noyau(x), R¹ représente un groupe hydrocarboné monovalent saturé ou insaturé ayant 1 à 20 atomes de carbone, et R représente un groupe hydrocarboné monovalent saturé ou insaturé ayant un groupe perfluoroalkyle et ayant 2 à 22 atomes de carbone, R étant un groupe représenté par la formule générale (2) suivante :
CₘF₂ₘ₊₁CₚH₂ₚ- (2)
dans laquelle m représente un nombre naturel de 2 à 16, et p représente un entier de 2 à 4,
et un liquide ionique.

5. Utilisation selon la revendication 4, dans laquelle Ar est un cycle condensé ayant un ou plusieurs cycles aromatiques hydrocarbonés, ou un groupe ayant une pluralité de cycles aromatiques liés les uns aux autres par une/des simple(s) liaison(s), un ou plusieurs des cycles aromatiques étant un cycle aromatique hydrocarboné.

6. Utilisation selon la revendication 4 ou 5, dans laquelle Ar est un groupe biphénylène.
